(19) Europäisches Patentamt
European Patent Office
Office européen des brevets

(11) Veröffentlichungsnummer: **0 183 927**
**B1**

(12) # EUROPÄISCHE PATENTSCHRIFT

(45) Veröffentlichungstag der Patentschrift: **13.09.89**

(51) Int. Cl.⁴: **C07C 50/06**, C07D 303/02, C07C 175/00

(21) Anmeldenummer: **85111584.0**

(22) Anmeldetag: **13.09.85**

(54) **Verfahren zur Herstellung optisch aktiver Chinonderivate.**

(30) Priorität: **11.10.84 CH 4871/84**

(43) Veröffentlichungstag der Anmeldung:
**11.06.86 Patentblatt 86/24**

(45) Bekanntmachung des Hinweises auf die Patenterteilung:
**13.09.89 Patentblatt 89/37**

(84) Benannte Vertragsstaaten:
**AT BE CH DE FR GB IT LI NL**

(56) Entgegenhaltungen:
**EP-A- 0 107 738**
**DE-A- 1 951 928**
**DE-A- 2 645 207**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE AG, Postfach 3255, CH-4002 Basel(CH)**

(72) Erfinder: **Rüttimann, August, Dr., General Guisan-Strasse 41, CH-4144 Arlesheim(CH)**

(74) Vertreter: **Cottong, Norbert A. et al, Grenzacherstrasse 124 Postfach 3255, CH-4002 Basel(CH)**

**EP 0 183 927 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von Chinonderivaten, welche als Zwischenprodukte für die Herstellung von (2RS,4'R,8'R)-Tocopherol geeignet sind. Die Erfindung betrifft ferner ein neues Ausgangs- und neue Zwischenprodukte in diesem Verfahren.

Das erfindungsgemässe Verfahren ist dadurch gekennzeichnet, dass man die Verbindung der Formel

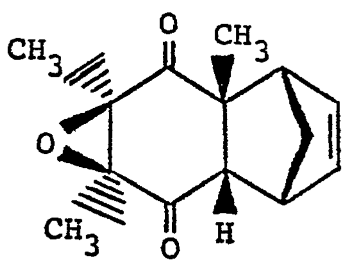

I

mit einer Verbindung der allgemeinen Formel

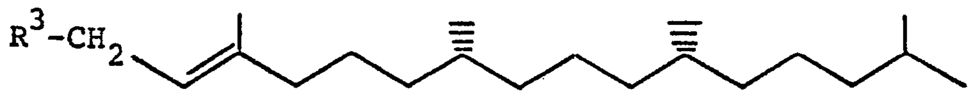

II

worin $R^3$ eine Abgangsgruppe darstellt, umsetzt, dass man, gewünschtenfalls, die so erhaltene Verbindung der Formel

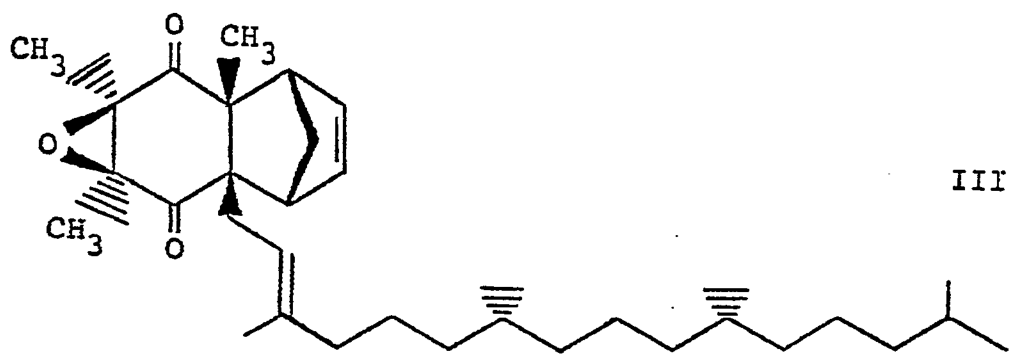

III

in die Verbindung der Formel

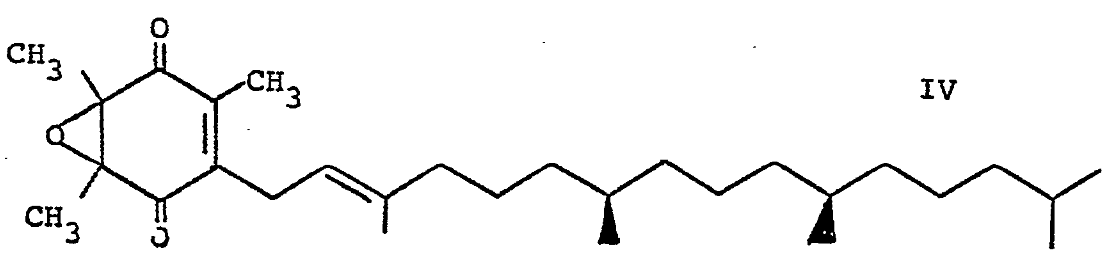

IV

überführt und dass man, gewünschtenfalls, die so erhaltene Verbindung der Formel IV in die Verbindung der Formel

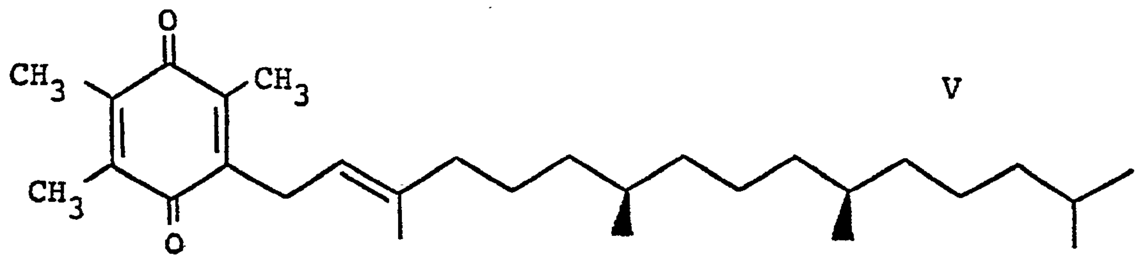

V

überführt.

Der Ausdruck "Abgangsgruppe" bedeutet im Rahmen der vorliegenden Erfindung insbesondere Halogen wie Fluor, Chlor, Brom und Jod, wobei Brom und Chlor bevorzugt sind, oder auch Gruppen wie die Mesylgruppe, die Tosylgruppe, die Acetat gruppe und dergleichen. Weiterhin bedeutet das Zeichen "◄", dass sich der entsprechende Rest oberhalb der Molekülebene befindet und das Zeichen ";k1;k1;k1", dass sich der entsprechende Rest unterhalb der Molekülebene befindet.

Die Verbindungen der Formeln I, III und IV sind neu und ebenfalls Gegenstand der vorliegenden Erfindung.

Die Umsetzung der Verbindung der Formel I mit einer Verbindung der allgemeinen Formel II kann in einem unter den Reaktionsbedingungen inerten organischen Lösungsmittel und in Gegenwart einer starken Base erfolgen. Als Lösungsmittel kommen hier in Frage sowohl polare als auch apolare Lösungsmittel. Bevorzugt sind apolare aprotische Lösungsmittel wie z.B. aliphatische oder aromatische Kohlenwasserstoffe wie Hexan, Benzol, Toluol und dergleichen und tert. Butanol als polares protisches Lösungsmittel. Ebenfalls bevorzugt sind Gemische dieser Lösungsmittel. Als starke Base kommen im Rahmen der vorliegenden Erfindung insbesondere in Frage organische Basen, wie z.B. Amide wie Alkalimetallamide (Li, Na, K) oder Lithiumdialkylamide, Alkoholate wie Alkalimetall-tert. Butylate oder auch Hydride wie Natrium- oder Kaliumhydrid und dergleichen. Die Reaktion kann ferner bei einer Temperatur von etwa -70°C bis etwa +50°C, vorzugsweise bei etwa 0°C bis etwa +30°C und insbesondere bei etwa Raumtemperatur erfolgen.

Die Ueberführung der Verbindung der Formel III in die Verbindung der Formel IV stellt eine Retro-Diels-Alder-Reaktion dar und kann in an sich bekannter Weise durchgeführt werden. Das Erhitzen kann in Abwesenheit oder in Gegenwart eines inerten Lösungsmittels erfolgen, beispielsweise bei einer Temperatur von etwa 140°C bis etwa 200°C, vorzugsweise bei einer Temperatur von etwa 150°C bis etwa 170°C.

Die Ueberführung der Verbindung der Formel IV in die Verbindung der Formel V, kann in an sich bekannter Weise durchgeführt werden. Zweckmässig erfolgt dies durch reduktive Abspaltung der Epoxygruppe. Als Reduktionsmittel sind besonders geeignet, Zink in Essigsäure, Zinkamalgam in Essigsäure, Natriumbisulfit, Lithium in Diäthylamin und dergleichen. Bevorzugte Reduktionssysteme sind Zink oder Zinkamalgam in Essigsäure. Da bei dieser Reduktion die Carbonylgruppen ebenfalls reduziert werden, muss das dabei anfallende Hydrochinon anschliessend wieder oxidiert werden. Diese Oxidation kann ebenfalls in an sich bekannter Weise durchgeführt werden. Als Oxidationsmittel können Sauerstoff, Luft, Javelle-Lauge, Alkalimetallchromate und dergleichen verwendet werden, wobei Sauerstoff (Luft) und Javelle-Lauge besonders geeignet sind.

Die Verbindung der Formel V ist bekannt und kann in bekannter Weise in (2RS,4'R,8'R)-Tocopherol übergeführt werden. Beispielsweise kann dies gemäss Isler, O. et. al., Vitamins and Hormones,20, 389 (1962) erfolgen.

Die in dem erfindungsgemässen Verfahren als Ausgangsmaterial verwendete Verbindung der Formel I ist neu. Sie kann jedoch leicht in an sich bekannter Weise durch Epoxidierung der Verbindung der Formel

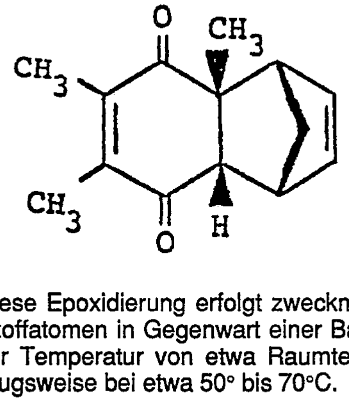

VI

erhalten werden. Diese Epoxidierung erfolgt zweckmässig mit 30%igem $H_2O_2$ in einem niederen Alkohol mit 1 bis 4 Kohlenstoffatomen in Gegenwart einer Base wie z.B. Alkali-und Erdalkalimetallcarbonate oder Hydroxide, bei einer Temperatur von etwa Raumtemperatur bis etwa Rückflusstemperatur des Reaktionsgemisches, vorzugsweise bei etwa 50° bis 70°C.

Beispiel 1

In einen 350 ml Sulfierkolben, versehen mit Rührer, Rückflusskühler und Argonbegasung, wurden unter Argon 180 ml eines Gemisches von tert.Butanol/Toluol (4:1) und 9,6 g (0,25 Mol) Kalium gegeben und während 2 Stunden am Rückfluss erhitzt. Hierauf wurde das Gemisch mittels eines Eisbades auf +3°C abgekühlt und mit 26,6 g (0,112 Mol) (1aRS,2aRS,3SR,6RS,6aSR,7aSR) -1a,2a,3,6,6a,7a-Hexahydro - 1a,6a,7a-trimethyl-3,6-methanooxireno[b]naphthalin -2,7-dion versetzt. Danach wurden 56,5 g (0,157 Mol) (2E,7R,11R)-Phytylbromid in 80 ml tert. Butanol/Toluol (4:1) innert 30 Minuten bei +5°C zugetropft und das Gemisch noch 30 Minuten bei Raumtemperatur nachgerührt. Anschliessend wurden 20 ml Wasser zugegeben und das Gemisch bei 30°C am Rotationsverdampfer eingeengt. Der Rückstand wurde zweimal in je 500 ml Hexan aufgenommen, zweimal mit halbgsättigter NaCl-Lösung gewaschen und anschliessend über $Na_2SO_4$ getrocknet. Man erhielt 68,3 g braunes Rohprodukt. Daraus wurden durch Chromatographie an Kieselgel mit Hexan/Aether (97:3) 40,2 g (1aRS,2aRS 3SR,6RS,6aSR,7aSR) - 1a,2a,3,6,6a,7a-Hexahydro-1a,6a,7a-trimethyl-2a-[(E,7R,11R)-3,7,11,15-tetramethyl-2-hexadecenyl] - 3,6-methanooxireno[b]naphthalin-2,7-dion als hellgelbes Oel mit einem HPLC-Gehalt von 99% erhalten.

Das als Ausgangsmaterial verwendete (1aRS,2aRS,3SR,6RS,6aSR,7aSR) -1a,2a,3,6,6a,7a-Hexahydro -1a,2a,7a,-trimethyl-3,6-methanooxireno[b]naphthalin -2,7-dion wurde wie folgt hergestellt:

In einem 2,5 Liter Sulfierkolben, versehen mit Rührer, Rückflusskühler unde Argonbegasung, wurden unter Argon 143,1 g (0,663 Mol) 1,4,4a,8aα-Tetrahydro-4aα,6,7-trimethyl -1α,4α-methanonaphthalin-5,8-dion in 1,1 Liter Aethanol bei 50°C gelöst. Dann wurde bei 50°C während 1,5 Stunden eine Lösung aus 23 g (0,3 Aeq.) $Na_2CO_3$ in 500 ml Wasser und 115 ml (1,5 Aeq.) 30%igem $H_2O_2$ zugetropft und das Gemisch während 30 Minuten bei 50°C nachgerührt. Anschliessend wurde auf Raumtemperatur abgekühlt,dreimal mit je 1 Liter Aether extrahiert, mit Wasser, verdünnter $NaHCO_3$-Lösung, gesättigter NaCl-Lösung gewaschen,dann getrocknet und eingeengt. Man erhielt 165,7 g weisse Kristalle.Nach Umkristallisation aus Methanol erhielt man 146,6 g (1aRs,2aRS,3SR,6RS,6aSR,7aSR) -1a,2a,3,6,6a,7a-Hexahydro-1a,2a,7a-trimethyl -3,6-methanooxireno[b]naphthalin-2,7-dion mit einem Schmelzpunkt von 72°-73°C.

Beispiel 2

In einem 150 ml Rundkolben wurden unter Argon 40,2 g (78,6 mMol) (1aRS,2aRS,3SR,6RS,6aSR,7aSR) -1a,2a,3,6,6a,7a-Hexahydro-1a,6a,7a- trimethyl -2a-[(E,7R,11R) - 3,7,11,15-tetramethyl-2-hexadecenyl] -3,6- methanooxireno[b]naphthalin-2,7-dion (hergestellt gemäss Beispiel 1) auf 170°C während 4 Stunden erhitzt. Hierauf wurde auf Raumtemperatur abgekühlt und an $SiO_2$ mit Essigsäureäthylester/Hexan (5:95) chromatographiert. Durch Einengen der reinen Eluate erhielt man 34,1 g (1SR,6RS)-1,4,6-Trimethyl -3-[(E,7R,11R)- 3,7,11,15-tetramethyl -2-hexadecenyl]-7-oxabicyclo[4.1.0]hept -3-en-2,5 dion als gelbes Oel mit einem HPLC-Gehalt von 99%.

Beispiel 3

In einem 500 ml Sulfierkolben, versehen mit Rührer, Rückflusskühler und Argonbegasung, wurden unter Argon 34,1 g (76,8 mMol) (1SR,6RS)-1,4,6-Trimethyl -3-[(E,7R,11R)-3,7,11,15-tetramethyl -2- hexadecenyl]-7-oxabicyclo[4.1.0]hept -3-en-2,5-dion (hergestellt gemäss Beispiel 2) in 350 ml Essigsäure gelöst und mit 20,5 g amalgamiertem Zink versetzt, wobei sich das Gemisch erhitzte. Nach 1,5 Stunden Rühren wurde zweimal mit 0,5 l Hexan extrahiert. Die vereinigten Extrakte wurden mit 3N-HCl und dann mit gesättigter $NaHCO_3$-Lösung gewaschen. Dann wurde die Hexanphase mit 50 ml 2N-NaOH versetzt und es wurde während 1 Stunde, unter Rühren, Sauerstoff durch das Gemisch geleitet. Anschliessend wurde das Gemisch zweimal mit je 0,5 l Hexan extrahiert, die Extrakte zweimal mit je 0,5 l Wasser gewaschen, getrocknet und am Wasserstrahlvakuum bei 40°C eingeengt.

Man erhielt 32,8 g rohes Produkt in Form eines gelben Oeles. Nach Chromatographie an $SiO_2$ mit Aether/Hexan (1:19) erhielt man 32,5 g 2,5,6-Trimethyl-3-[(E,7R,11R) -3,7,11,15-tetramethyl-2- hexadecenyl]-benzochinon mit einem HPLC-Gehalt von 99%.

**Patentansprüche für die Vertragsstaaten: BE, CH, DE, FR, GB, IT, LI, NL**

1. Verfahren zur Herstellung von Chinonderivaten, dadurch gekennzeichnet, dass man die Verbindung der Formel

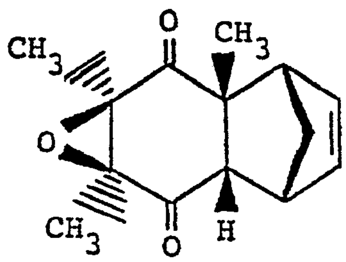

I

mit einer Verbindung der allgemeinen Formel

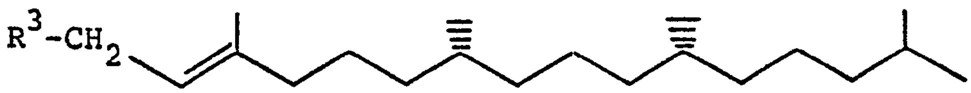

II

worin $R^3$ eine Abgangsgruppe darstellt, umsetzt, dass man, gewünschtenfalls, die so erhaltene Verbindung der Formel

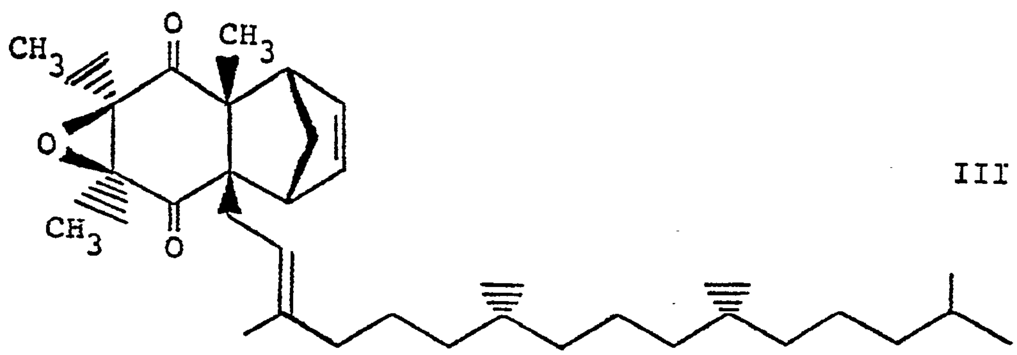

III

in die Verbindung der Formel

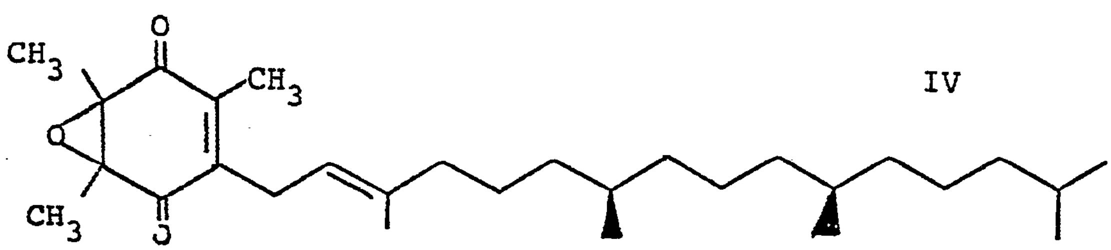

IV

überführt und dass man, gewünschtenfalls, die so erhaltene Verbindung der Formel IV in die Verbindung der Formel

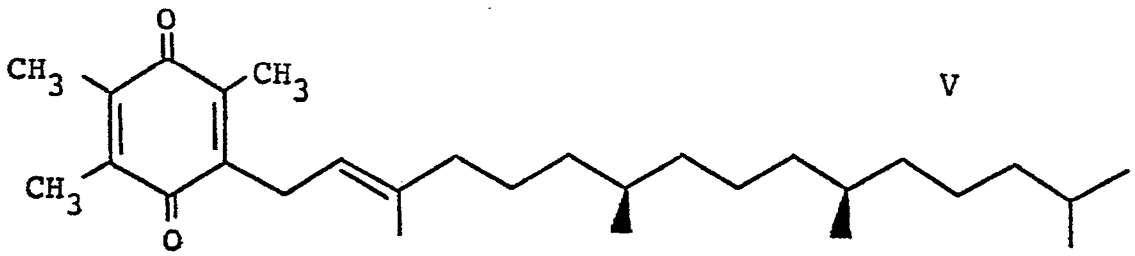

V

überführt.

2. Verfahren gemäss Anspruch 1, dadurch gekennzeichnet, dass man die Umsetzung der Verbindung der Formel I mit einer Verbindung der Formel II in einem inerten, organischen Lösungsmittel und in Gegenwart einer starken Base durchführt.

3. Verfahren gemäss Anspruch 2, dadurch gekennzeichnet, dass man als inertes, organisches Lösungsmittel ein apolares aprotisches, ein polares protisches Lösungsmittel oder Gemische hiervon verwendet.

4. Verfahren gemäss Anspruch 2 oder 3, dadurch gekennzeichnet dass man als starke Base ein Alkalimetallamid, ein Lithiumdialkylamid oder ein Alkalimetall-tert.Butylat verwendet.

5. Verfahren gemäss einem der Ansprüche 1-4, dadurch gekennzeichnet, dass man die Umsetzung der Verbindung der Formel I mit einer Verbindung der Formel II bei einer Temperatur von etwa -70°C bis etwa +50°C, vorzugsweise bei etwa 0°C bis etwa +30°C und insbesondere bei etwa Raumtemperatur durchführt.

6. Die Verbindung der Formel

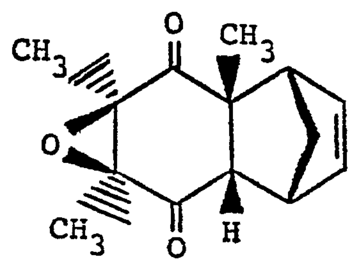

I

7. Die Verbindung der Formel

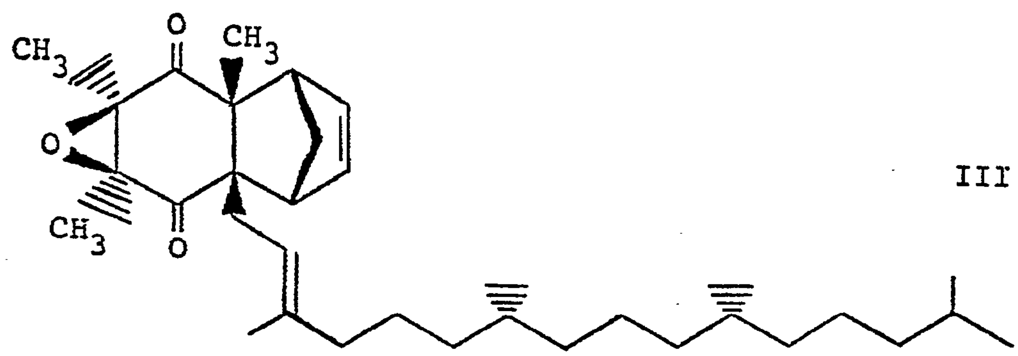

III

8. Die Verbindung der Formel

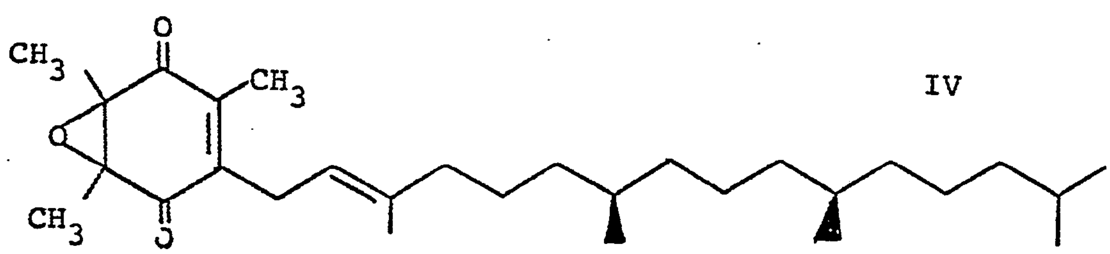

IV

**Patentansprüche für den Vertragsstraat: AT**

1. Verfahren zur Herstellung von Chinonderivaten, dadurch gekennzeichnet, dass man die Verbindung der Formel

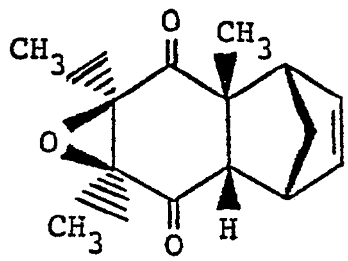

I

mit einer Verbindung der allgemeinen Formel

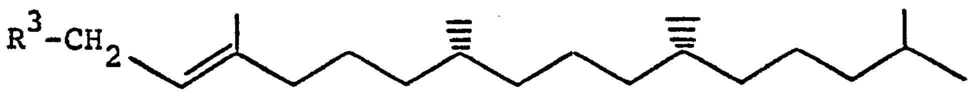

II

worin $R^3$ eine Abgangsgruppe darstellt, umsetzt, dass man, gewünschtenfalls, die so erhaltene Verbindung der Formel

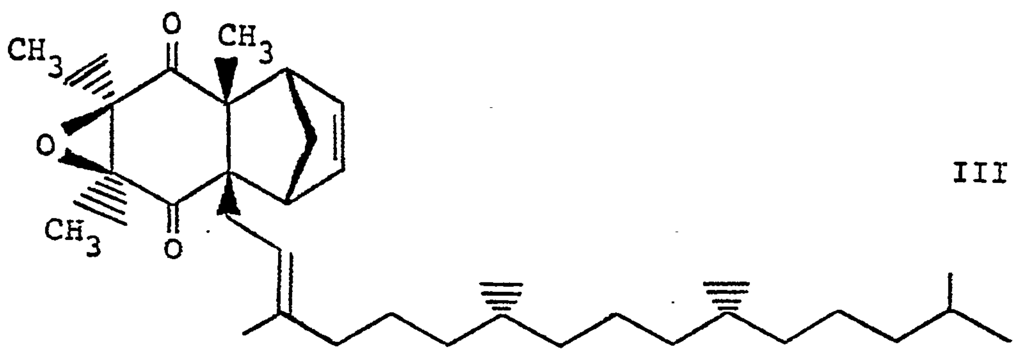

III

in die Verbindung der Formel

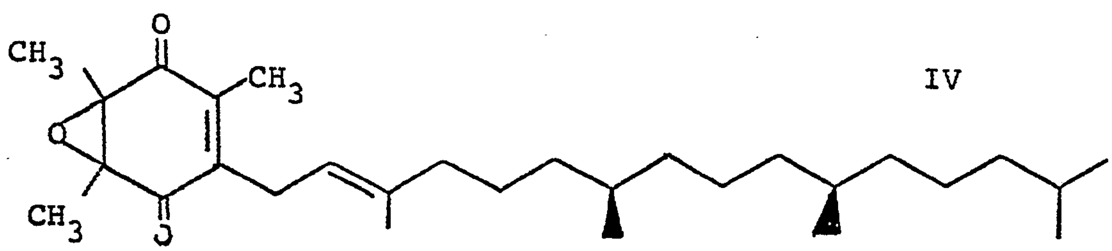

IV

überführt und dass man, gewünschtenfalls, die so erhaltene Verbindung der Formel IV in die Verbindung der Formel

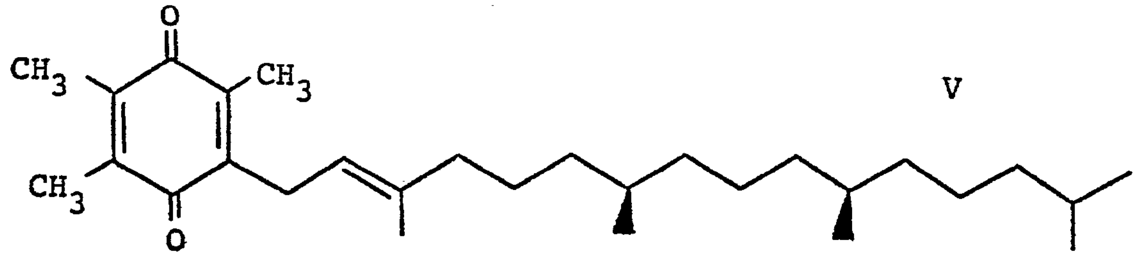

V

überführt.

2. Verfahren gemäss Anspruch 1, dadurch gekennzeichnet, dass man die Umsetzung der Verbindung der Formel I mit einer Verbindung der Formel II in einem inerten, organischen Lösungsmittel und in Gegenwart einer starken Base durchführt.

3. Verfahren gemäss Anspruch 2, dadurch gekennzeichnet, dass man als inertes, organisches Lösungsmittel ein apolares aprotisches, ein polares protisches Lösungsmittel oder Gemische hiervon verwendet.

4. Verfahren gemäss Anspruch 2 oder 3, dadurch gekennzeichnet, dass man als starke Base ein Alkalimetallamid, ein Lithiumdialkylamid oder ein Alkalimetall-tert.Butylat verwendet.

5. Verfahren gemäss einem der Ansprüche 1-4, dadurch gekennzeichnet, dass man die Umsetzung der Verbindung der Formel I mit einer Verbindung der Formel II bei einer Temperatur von etwa -70°C bis etwa +50°C, vorzugsweise bei etwa 0°C bis etwa +30°C und insbesondere bei etwa Raumtemperatur durchführt.

**Claims for the Contracting States BE, CH, DE, FR, GB, IT, LI, NL**

1. A process for the manufacture of quinone derivatives, characterized by reacting the compound of the formula

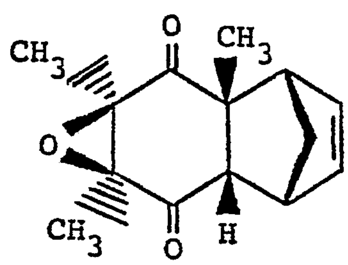

I

with a compound of the general formula

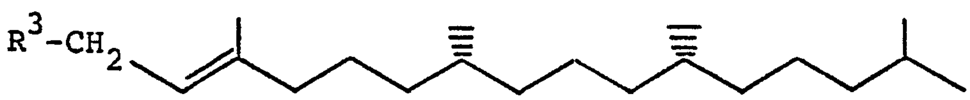

II

wherein $R^3$ represents a leaving group, if desired, converting the thus obtained compound of the formula

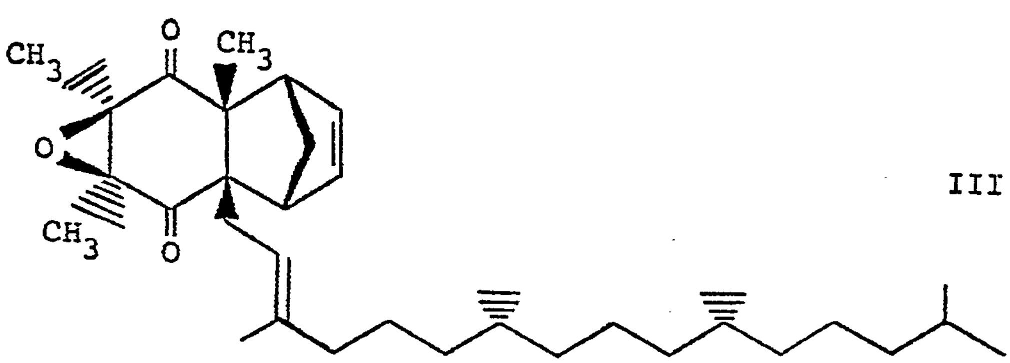

III

into the compound of the formula

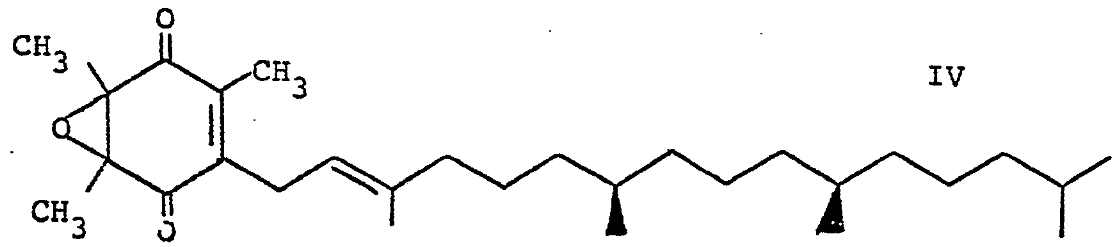

IV

and, if desired, converting the thus- obtained compound of formula IV into the compound of the formula

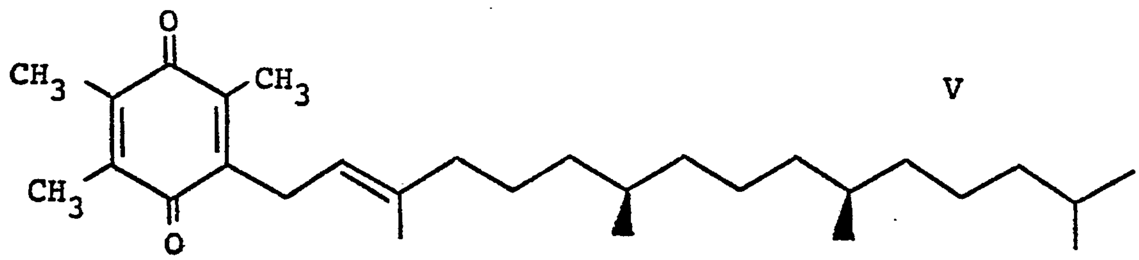

V

2. A process according to claim 1, characterized in that the reaction of the compound of formula I with a compound of formula II is carried out in an inert, organic solvent and in the presence of a strong base.

3. A process according to claim 2, characterized in that an apolar aprotic solvent, a polar protic solvent or a mixture thereof is used as an inert, organic solvent.

4. A process according to claim 2 or 3, characterized in that an alkali metal amide, a lithium dialkylamide or an alkali metal tert. butylate is used as a strong base.

5. A process acording to any one of claims 1–4, characterized in that the reaction of the compound of formula I with a compound of formula II is carried out at a temperature of about –70°C to about +50°C, preferably at about 0°C to about +30°C and especially at about room temperature.

6. The compound of the formula

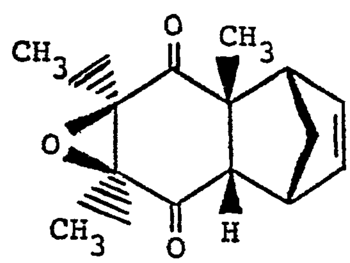

I

7. The compound of the formula

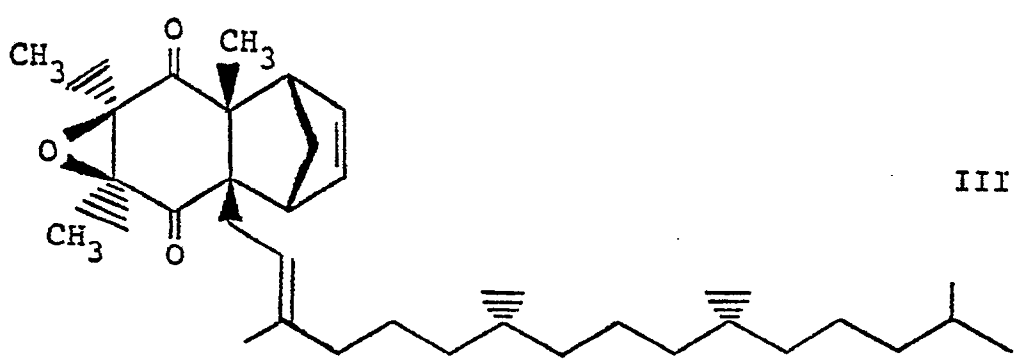

III

8. The compound of the formula

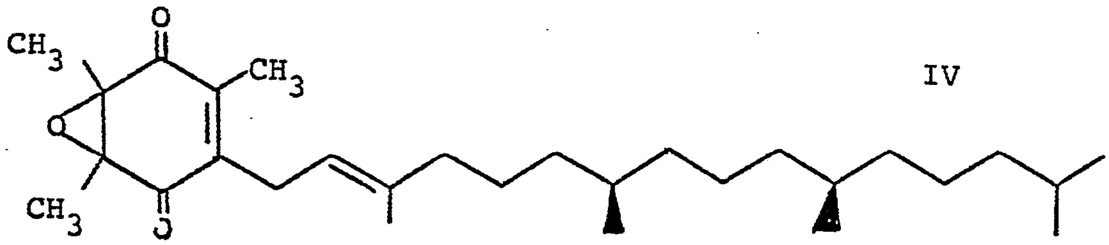

IV

**Claims for the Contracting State AT**

1. A process for the manufacture of quinone derivatives, characterized by reacting the compound of the formula

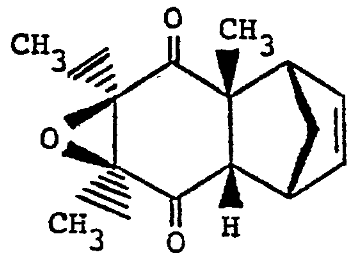

I

with a compound of the general

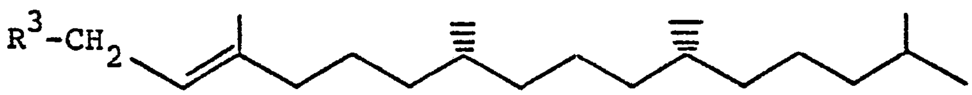

II

wherein $R^3$ represents a leaving group, if desired, converting the thus-obtained compound of the formula

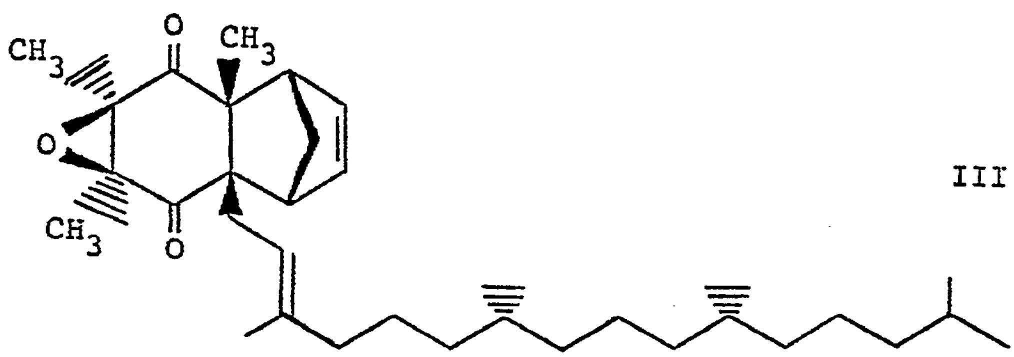

III

into the compound of the formula

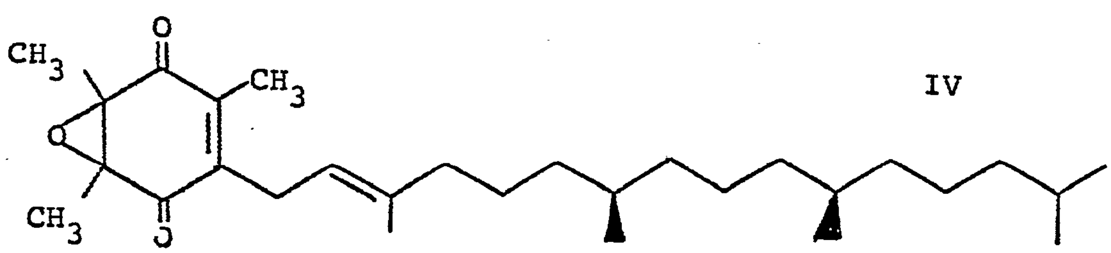

IV

and, if desired, converting the thus- obtained compound of formula IV into the compound of the formula

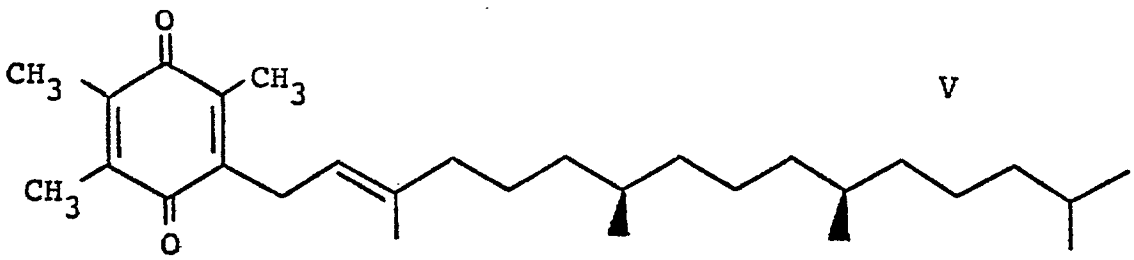

V

2. A process according to claim 1, characterized in that the reaction of the compound of formula I with a compound if formula II is carried out in an inert, organic solvent and in the presence of a strong base.

3. A process according to claim 2, characterized in that an apolar aprotic solvent, a polar protic solvent or a mixture thereof is used as the inert, organic solvent.

4. A process according to claim 2 or 3, characterized in that an alkali metal amide, a lithium dialkylamide or an alkali metal tert. butylate is used as a strong base.

5. A process according to any one of claims 1–4, characterized in that the reaction of the compound of formula I with a compound of formula II is carried out at a temperature of about –70°C to +50°C, preferably at about 0°C to about +30°C and especially at about room temperature.

**Revendications pour les Etats contractants: BE, CH, DE, FR, GB, IT, LI, NL**

1. Procédé de préparation de dérivés de la quinone, caractérisé en ce que l'on fait réagir le composé de formule

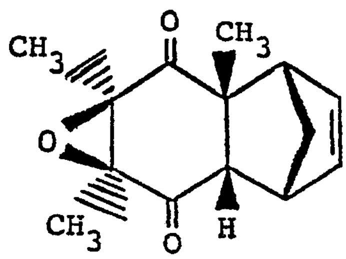

I

avec un composé de formule générale

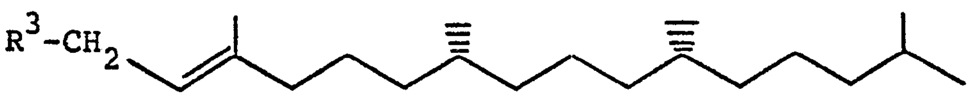

II

dans laquelle $R^3$ représente un groupe éliminable, en ce que, si on le désire, on convertit le composé ainsi obtenu, répondant la formule

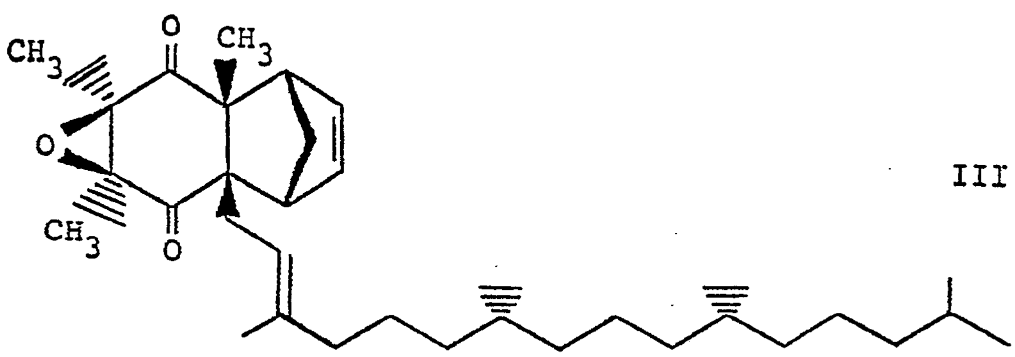

III

en le composé de formule

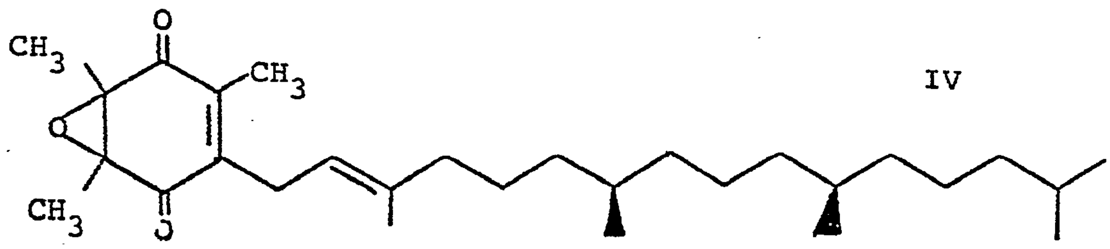

IV

et en ce que, si on le désire, on convertit le composé de formule IV ainsi obtenu en le composé de formule

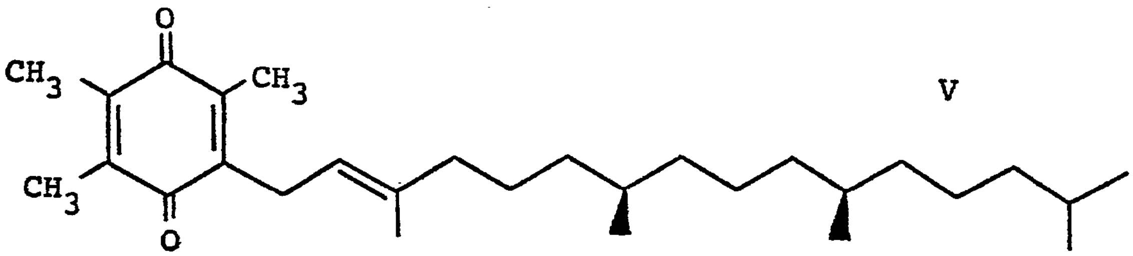

V

2. Procédé selon la revendication 1, caractérisé en ce que la réaction entre le composé de formule I et un composé de formule II est effectuée dans un solvant organique inerte en présence d'une base forte.

3. Procédé selon la revendication 2, caractérisé en ce que l'on utilise en tant que solvant organique inerte un solvant apolaire aprotonique, un solvant polaire protonique ou un mélange de tels solvants.

4. Procédé selon la revendication 2 ou 3, caractérisé en ce que l'on utilise en tant que base forte un amidure de métal alcalin, un dialkylamidure de lithium ou un tert-butylate de métal alcalin.

5. Procédé selon l'une des revendications 1 à 4, caractérisé en ce quel'on effectue la réaction entre le composé de formule I et un composé de formule II à une température allant d'environ –70°C à +50°C, de préférence d'environ 0°C à +30°C et plus spécialement au voisinage de la température ambiante.

6. Le composé de formule

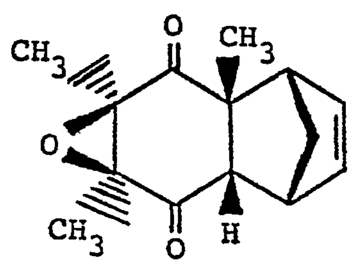

I

7. Le composé de formule

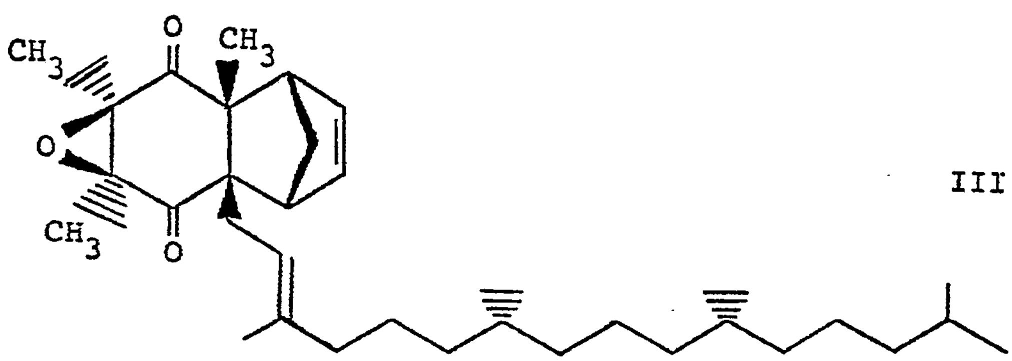

III

8. Le composé de formule

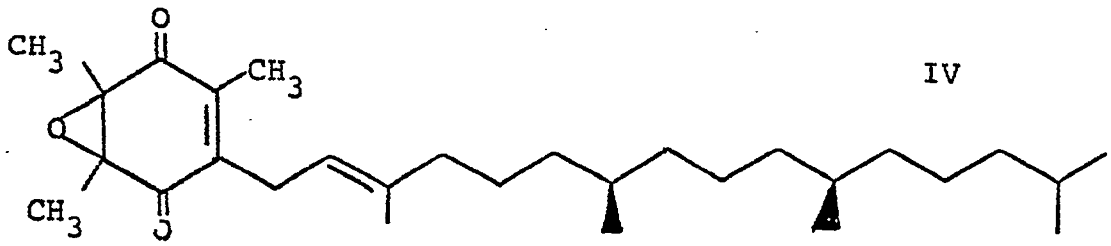

IV

**Revendications pour l'Etat contractant: AT**

1. Procédé de préparation de dérivés de la quinone, caractérisé en ce que l'on fait réagir le composé de formule

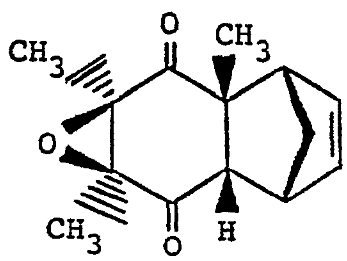

I

avec un composé de la formule générale

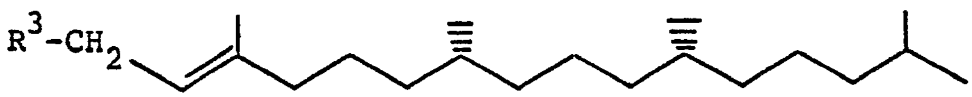

II

dans laquelle $R^3$ représente un groupe éliminable, en ce que, si on le désire, on convertit le composé ainsi obtenu, répondant à la formule

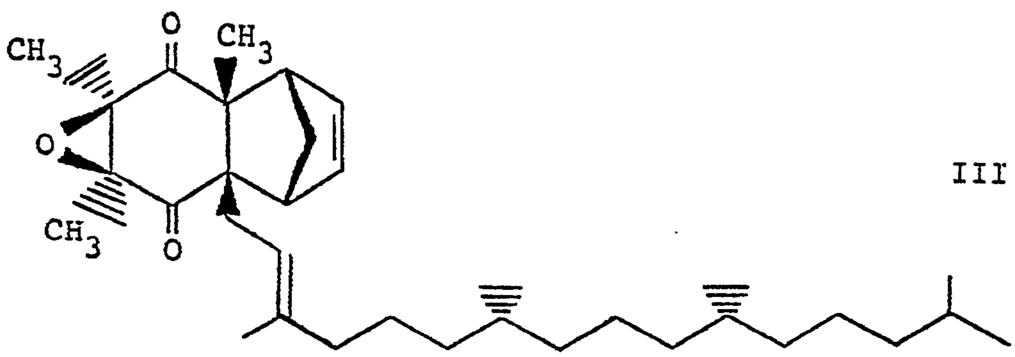

III

en le composé de formule

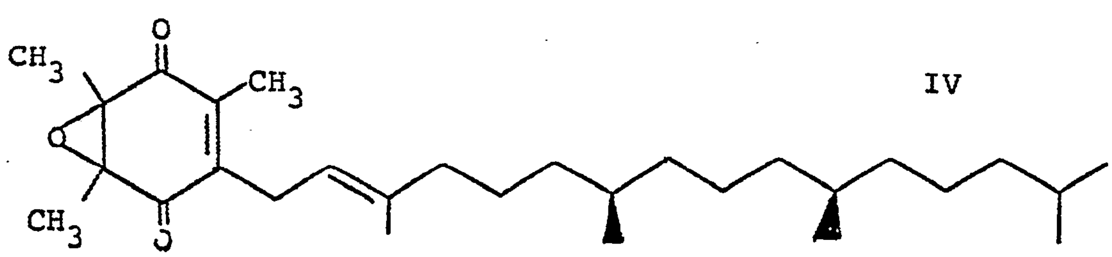

IV

et en ce que, si on le désire, on convertit le composé de formule IV ainsi obtenu en le composé de formule

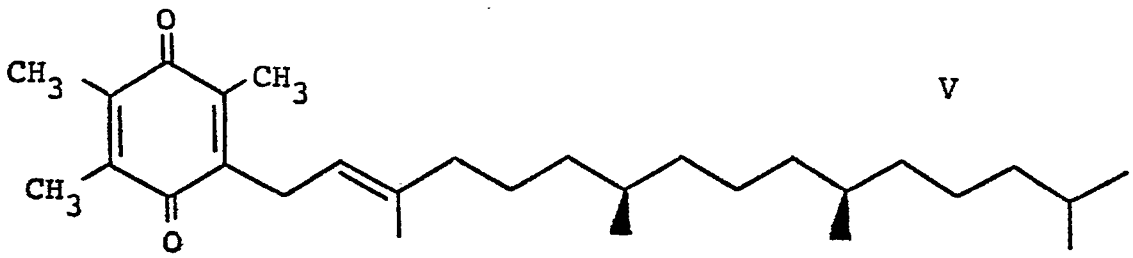

V

2. Procédé selon la revendication 1, caractérisé en ce que la réaction entre le composé de formule I et un composé de formule II est effectuée dans un solvant organique inerte en présence d'une base forte.

3. Procédé selon la revendication 2, caractérisé en ce que l'on utilise en tant que solvant organique inerte un solvant apolaire aprotonique, un solvant polaire protonique ou un mélange de tels solvants.

4. Procédé selon la revendication 2 ou 3, caractérisé en ce que l'on utilise en tant que base forte un amidure de métal alcalin, un dialkylamidure de lithium ou un tert-butylate de métal alcalin.

5. Procédé selon l'une des revendications 1 à 4, caractérisé en ce que l'on effectue la réaction entre le composé de formule I et un composé de formule II à une température allant d'environ –70°C à +50°C, de préférence d'environ 0°C à +30°C et plus spécialement au voisinage de la température ambiante.